# EUROPÄISCHE PATENTSCHRIFT

(11) **EP 0 713 880 B1**
(45) Veröffentlichungstag und Bekanntmachung des Hinweises auf die Patenterteilung: **10.06.1998**
(21) Anmeldenummer: 95117386.3
(22) Anmeldetag: 04.11.1995
(51) Int. Cl.: C07J 53/00, C07J 1/00

(54) **Verfahren zur stereoselektiven Reduktion von 17-Oxo-Steroiden**
Stereoselective reduction of 17-ketosteroids
Procédé de réduction stéréospécifique des stéroides 17-oxo

(30) Priorität: 22.11.1994 DE 4441462
(43) Veröffentlichungstag der Anmeldung: 29.05.1996
(73) Patentinhaber: Jenapharm GmbH & Co. KG, 07745 Jena (DE)
(72) Erfinder: Schwarz, Sigfrid, Prof. Dr., D-07743 Jena (DE); Martens, Jürgen, Prof. Dr., D-26122 Oldenburg (DE); Reiners, Iris, D-26131 Oldenburg (DE); Trentmann, Wilhelm G., D-26122 Oldenburg (DE); Wilken, Jörg, D-26135 Oldenburg (DE); Mehler, Thomas, D-26129 Oldenburg (DE); Daulsberg, Christine, D-26121 Oldenburg (DE); Behnen, Willi, D-26129 Oldenburg (DE)
(74) Vertreter: Cramer, Eva-Maria

(56) Entgegenhaltungen:
- EP-A- 0 142 566
- EP-A- 0 171 175
- FR-A- 2 429 797
- TETRAHEDRON: ASYMMETRY, Bd. 3, Nr. 12, 1992, OXFORD GB, Seiten 1475-1504, XP002001067 S. WALLBAUM ET AL: "Asymmetric Syntheses with Chiral Oxazaborolidines"
- JOURNAL OF THE CHEMICAL SOCIETY, PERKIN TRANSACTIONS 1, Nr. 10, Oktober 1985, LETCHWORTH GB, Seiten 2039-2044, XP002001068 S. ITSUNO ET AL: "Asymmetric Syntheses Using Chirally Modified Borohydrides. Part 3. Enantioselective Reduction of Ketones and Oxime Ethers with Reagents Prepared from Borane and Chiral Amino Alcohols"
- DATABASE WPI Section Ch, Week 8242 Derwent Publications Ltd., London, GB; Class B03, AN 82-89115E XP002001071 & JP-A-57 146 786 (KYOWA HAKKO KOGYO KK) , 10.September 1982
- JOURNAL FÜR PRAKTISCHE CHEMIE, Bd. 328, Nr. 1, 1986, LEIPZIG, DE, Seiten 55-70, XP002001069 R. PROUSA ET AL:
- PHARMAZIE, Bd. 34, Nr. 4, 1979, BERLIN DE, Seite 250 XP002001070 K. PONSOLD ET AL: "Synthese von 14,15-ungesättigten Östratrien-3-methylethern"

## Beschreibung

Es wird ein neues Verfahren zur stereoselektiven Reduktion von 17-Oxo-Steroiden der allgemeinen Formel I 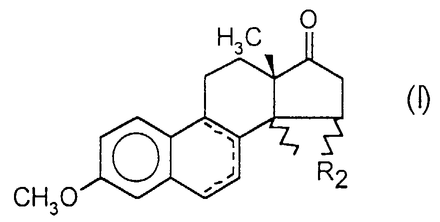 in der das Symbol 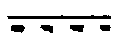 eine Einfach- oder eine Doppelbindung bedeutet und R₂ entweder eine CH₂-Gruppe oder eine weitere Bindung zwischen den C-Atomen 14 und 15 darstellt, beschrieben.

Das erfindungsgemäße Verfahren eignet sich zur Synthese von Steroidwirkstoffen, die eine 14,15-Methylengruppe enthalten.

Die Patentschrift DD 145 919 beschreibt 14α,15α-Methylen-17β-estradiol-3-methylether, 14α,15α-Methylen-17α-estradiol-3-methylether, 14β,15β-Methylen-17β-estradiol-3-methylether und 14β,15β-Methylen-17α-estradiol-3-methylether als Wirkstoffe, die für die orale Kontrazeption geeignet sind.
Ester des 14α,15α-Methylen-estra-1,3,5(10)-trien-3,17β-diol sind oral wirksame Estrogene, die 17α-Ethinylestradiol als bisher gebräuchlichstes Estrogen hinsichtlich Wirkungsstärke und Wirkungsverlauf deutlich übertreffen (P 43 22 186.6).
14α,15α-Methylen-und 14β,15β-Methylen-dihydroequilenin sind als erfolgversprechende Zielstrukturen D-Ring modifizierter Steroidhormone beschrieben (Tetrahedron Letters 35 (1994) 2329 - 2330).

Für die Herstellung dieser Wirkstoffe sind in der Fach- und Patentliteratur Verfahren beschrieben, die sich entweder auf 14-Dehydroestron-3-methylether zurückführen lassen (DD 145 919) oder von 3-Methoxy-estra-1,3,5(10),8,14-pentaen-17α-ol ausgehen (P 42 39 945.9; P 42 39 946.7; Tetrahedron Letters 35 (1994) 2329 - 2330).

In diesen Verfahren stellen Prozesse zur Reduktion von 17-Oxo-Steroiden, die als Vor- und Zwischenprodukte anfallen, Schlüsselreaktionen dar, die jedoch mit geringen bis sehr geringen Stereoselektivitäten verlaufen.
So wird der für das Verfahren nach PS DD 145 919 erforderliche 14-Dehydro-17α-estradiol-3-methylether durch Reduktion von 14-Dehydro-estron-3-methylether nach Chromatographie in einer Ausbeute von nur 10 % erhalten (Pharmazie 34 (1979) 250).
Die im Rahmen des gleichen Verfahrens notwendige Reduktion von 14α,15α-Methylen-estron-methylether führt nach jeweils chromatographischer Trennung mit Natriumborhydrid zu 45 % des 17α- und zu 55 % des 17β-Alkohols, mit Litiumaluminiumhydrid zu 26 % des 17α- und 60 % des 17β-Alkohols und mit Boran zu 17 % des 17α- und 67 % des 17β-Alkohols.

Die Reduktion des 14β,15β-Methylen-estron-methylethers, die mit Natriumborhydrid vorgenommen wird, ergibt nach chromatographischer Trennung 23 % des 17β- und 52.5 % des 17α-Alkohols.

Wird die Reduktion mit Boran durchgeführt, erhält man 8 % des 17β- und 72 % des 17α-Alkohols (J. prakt. Chem. 328 (1986) 55 - 70).

Die vom 3-Methoxy-estra-1,3,5(10),8,14-pentaen-17α-ol ausgehenden Ver-fahren führen bei der Reduktion des 3-Methoxy-14α,15α-methylenestra-1,3,5(10),8-tetraen-17-on mit Natriumborhydrid/CeCl₃ 7H₂O zu einem Verhältnis von 17α- : 17β- Alkohol wie 1 : 1,6 und bei der Reduktion von 14α,15α-Methylen-dihydroequilenin-methylether zu 37 % des 17α- und 59 % des 17β-Alkohols (Tetrahedron Letters 35 (1994) 2329 - 2330).

Der Nachteil dieser Lösungen besteht darin, daß die mangelhafte Stereoselektivität dieser Reduktionsschritte zwangsläufig zu Gemischen der 17α- und 17β-Alkohole führt, die mit chromatographischen Mitteln zu trennen sind.
Die anfallenden 17-Hydroxyverbindungen, die nicht benötigt werden, müssen reoxidiert und durch erneute Reduktion einer weiteren Nutzung zugeführt werden.
Daher sind alle bisherigen Verfahren zur Herstellung von 14,15-Methylen-Steroiden sehr aufwendig.

Aufgabe der Erfindung ist das Auffinden eines neuen Verfahrens zur vollständigen stereoselektiven Reduktion von 17-Oxo-Steroiden zu entsprechenden 17α- oder 17β-Alkoholen in hoher distereomerer Reinheit, wodurch, verglichen mit den bisher bekannten Reduktionsverfahren, eine wesentlich weniger aufwendige Herstellung von Steroidhormonen mit einer 14,15-Methylengruppe erreicht wird.

Erfindungsgemäß wird die Aufgabe dadurch gelöst, daß ein Verfahren zur stereoselektiven Reduktion von 17-Oxo-Steroiden entwickelt wurde, bei welchem man die Ketogruppe von 17-Oxo-Steroiden der allgemeinen Formel I 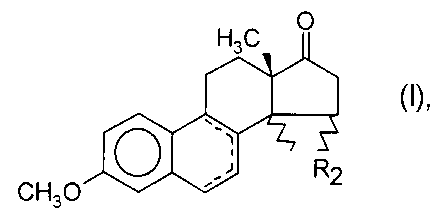 in der das Symbol 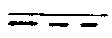 eine Einfach- oder eine Doppelbindung bedeutet und R₂ entweder eine CH₂-Gruppe oder eine weitere Bindung zwischen den C-Atomen14 und 15 darstellt, mit Boran oder Catecholboran zu entsprechenden 17α- oder 17β-Alkoholen in Gegenwart von homochiralen 1,2-Aminoalkoholen-Katalysatoren, reduziert.

Beispiele für solche Katalysatoren in der Reduktion von Ketonen mit Boran oder Catecholbran sind 1,3,2-Oxazaborolidine und 1,3,2-Oxazaphospholidine, die nach literaturbekannten Methoden [Tetrahedron: Asymmetry, Band 3, Seiten 1475 bis 1504 (1992); New J. Chem., Band 11, Seiten 573 bis 579 (1987)] vorher erzeugt und isoliert oder *in situ* aus folgenden 1,2-Aminoalkoholen gewonnen werden:
2-Aminoethanol,
2-Amino-2-methyl-1-propanol,
(*S*)-2-(Diphenylhydroxymethyl)azetidin,
(*R*)-2-(Diphenylhydroxymethyl)azetidin,
(*S*)-2-(Diphenylhydroxymethyl)pyrrolidin,
(*S*)-2-[Di(β-naphthyl)hydroxymethyl]pyrrolidin,
(*R*)-2-(Diphenylhydroxymethyl)pyrrolidin,
(*RS*)-2-(Diphenylhydroxymethyl)pyrrolidin,
(*R*)-(1,2,3,4-Tetrahydroisochinolin-3-yl)methanol,
(*R*)-Diphenyl-(3-thiazolin-4-yl)methanol,
(*R*)-(1,1-Dioxo-[1,2]thiazetidin-3-yl)diphenyl-methanol,
(*RS*)-Diphenyl-(3-thiazolin-4-yl)methanol,
(*RS*)-2-Amino-1,1-diphenyl-4-(methylsulfanyl)butan-1-ol,
(*R*)-2-Amino-1,1-diphenyl-3-(isopropylsulfanyl)propan-1-ol,
(*RS*)-2-Amino-1,1-diphenyl-3-(isopropylsulfanyl)propan-1-ol,
(*R*)-1-(1'-Amino-1'-phenylmethyl)cyclopentanol,
(*S*)-1-(1'-Amino-1'-phenylmethyl)cyclopentanol,
(*1'S*,*2'S*)-1-(1'-Amino-2'-methylbutyl)cyclopentanol,
(*R,R*)-2,2'-Ethyldiiminobis(1-butanol)

Besonders vorteilhaft für die Erfindung erweisen sich die in den Ansprüchen 2 bis 5 aufgezeigten Merkmale.

Es wurde überraschenderweise gefunden, daß der sterische Verlauf der Reduktion der 17-Ketogruppe, der ohne Zusatz eines Katalysators nur von der räumlichen Umgebung der Carbonylgruppe abhängt, in Gegen-wart eines Katalysators von dessen Chiralität beeinflußt wird.

So ist es möglich, bei gleicher stereochemischer Ausgangslage eines 17-Oxo-Steroids die Stereoselektivität der Reduktion mit Boran oder Catecholboran entweder vollständig in die Richtung des betreffenden 17α-Alkohols oder vollständig in die Richtung des 17β-Alkohols zu lenken, je nachdem, welches Enantiomer eines Katalysators zum Einsatz kommt.

Damit besteht die Möglichkeit, 17-Oxo-Steroide der allgemeinen Formel I gezielt stereoselektiv zu reduzieren.

Das erfindungsgemäße Verfahren eignet sich in besonderem Maße zur Herstellung von 14α,15α-Methylen-17β-estradiol-3-methylether, 14β,15β-Methylen-17β-estradiol-3-methylether, 3-Methoxy-14α,15α-methylen-estra-1,3,5(10),8-tetraen-17β-ol, 14α,15α-Methylen- dihydroequilenin-3-methylether und 17α-Estradiol-3-methyl ether aus den entsprechenden 17-Oxo-Steroiden.

Der Vorteil des erfindunggemäßen Verfahrens besteht darin, daß bei der Reduktion von 17-Oxo-Steroiden der allgemeinen Formel I mit Boran oder Catecholboran in Gegenwart von Katalysatoren, die sich von 1,2-Aminoalkoholen ableiten, gezielt 17α-Hydroxy- oder 17β-Hydroxy-Steroide in hoher diastereomerer Reinheit erhalten werden. Dadurch vermeidet man aufwendige Trennungen von Gemischen aus 17α- / 17β- Alkoholen und ein Recycling unerwünschter Abprodukte. Mit Hilfe des erfindungsgemäßen Verfahrens gelingt es, das bisherige Herstellungsverfahren für 14,15-Methylen-Steroide wesentlich zu verbessern.

Die Erfindung soll durch die nachfolgenden Ausführungsbeispiele näher erläutert, jedoch nicht eingeschränkt werden

### Beispiel 1

### Allgemeine Arbeitsvorschrift zur Reduktion von 17-Oxo-Steroiden der allgemeinen Formel I

Unter Argonatmosphäre werden 0,135 mmol (10 Mol%) 1,2-Aminoalkohol in 3 ml THF gelöst und mit einem PE 40/60/fl. N₂-Kältebad auf -70 °C gekühlt. Bei dieser Temperatur gibt man 1,5 ml einer 1 M BH₃-THF-Komplexlösung in THF (1,5 mmol) zu, läßt auf Raumtemperatur erwärmen und rührt eine weitere Stunde. Zu der auf 30 °C erwärmten Lösung gibt man innerhalb einer Stunde 1,35 mmol des Steroidketons in 3 ml THF und rührt 48 h bei dieser Temperatur. Zur Hydrolyse werden langsam 10 ml 2 N HCI unter Eiskühlung zugetropft. Nach der Phasentrennung wird die wäßrige Phase dreimal mit je 4 ml Methyl-tert. butylether extrahiert. Die vereinigten etherischen Phasen werden mit 5 ml wäßriger 2 N NaOH, zweimal mit je 5 ml ges. wäßriger NaCl-Lösung ausgeschüttelt und über wasserfreiem MgSO₄ getrocknet. Nach dem Abziehen des Lösungsmittels erhält man einen weißen Feststoff. Die Bestimmung des Diastereomerenverhältnisses erfolgt über eine HPLC-Analyse der Rohprodukte. Die Weiterverarbeitung der Reduktionsprodukte kann als Rohprodukt oder nach Umkristallisation erfolgen.

### Beispiel 2

### 14α,15α-Methylen-17β-estradiol-3-methylether

Entsprechend Beispiel 1 wird 14α,15α-Methylen-estronmethylether mit Boran in Gegenwart des sich vom 1,2-Aminoalkohol (*R*)-1-(1'-Amino-1'-phenylmethyl)cyclopentanol ableitenden Katalysators reduziert.
Laut HPLC-Analyse beträgt das Verhältnis 17β-Hydroxy-Steroid : 17α-Hydroxy-Steroid = 99 : 1.
Nach Aufarbeitung und Umkristallisation aus Methanol erhält man 14α,15α-Methylen-17β-estradiol-3-methylether: Fp.124 -127 °C.

### Beispiel 3

### 14α, 15α-Methylen-17β-estradiol-3-methylether

Entsprechend Beispiel 2 wird 14α,15α-Methylen-estronmethylether mit Boran in Gegenwart des sich vom 1,2-Aminoalkohol (*R*)-1-(1'-Amino-1'-phenylmethyl)cyclopentanol ableitenden Katalysators reduziert, wobei die Reduktion statt bei 30 °C bei 20 °C durchgeführt wurde.
Laut HPLC-Analyse beträgt das Verhältnis 17β-Hydroxy-Steroid : 17α-Hydroxy-Steroid = 99 : 1.
Nach Aufarbeitung und Umkristallisation aus Methanol erhält man 14α,15α-Methylen-17β-estradiol-3-methylether: Fp.122 -123 °C.

### Beispiel 4

### 14α,15α-Methylen-17β-estradiol-3-methylether

Entsprechend Beispiel 2 wird 14α,15α-Methylen-estronmethylether mit Boran in Gegenwart des sich vom 1,2-Aminoalkohol (*R*)-1-(1'-Amino-1'-phenylmethyl)cyclopentanol ableitenden Katalysators reduziert, wobei 5 Mol% statt 10 Mol% 1,2-Aminoalkohol eingesetzt wurden.
Laut HPLC-Analyse beträgt das Verhältnis 17β-Hydroxy-Steroid : 17α-Hydroxy-Steroid = 98 : 2.
Nach Aufarbeitung und Umkristallisation aus Methanol erhäelt man 14α,15α-Methylen-17β-estradiol-3-methylether: Fp.124 -126 °C.

### Beispiel 5

### 14α,15α-Methylen-17β-estradiol-3-methylether

Entsprechend Beispiel 4 wird 14α,15α-Methylen-estronmethylether mit Boran in Gegenwart des sich vom 1,2-Aminoalkohol (*R*)-1-(1'-Amino-1'-phenylmethyl)cyclopentanol ableitenden Katalysators reduziert, wobei 15 Mol% statt 10 Mol% 1,2-Aminoalkohol sowie statt 1,5 ml 1M BH₃-THF-Komplexlösung in THF nun 1,6 ml dieser Lösung eingesetzt wurden.
Laut HPLC-Analyse beträgt das Verhältnis 17βHydroxy-Steroid : 17α-Hydroxy-Steroid = 99 : 1.
Nach Aufarbeitung und Umkristallisation aus Methanol erhält man 14α,15α-Methylen-17β-estradiol-3-methylether: Fp.122 - 125 °C.

### Beispiel 6

### 14α,15α-Methylen-17β-estradiol-3-methylether

Entsprechend Beispiel 1 wird 14α,15α-Methylen-estronmethylether mit Boran in Gegenwart des sich vom 1,2-Aminoalkohol (*R*)-2-(Diphenylhydroxymethyl)pyrrolidin ableitenden Katalysators reduziert.
Laut HPLC-Analyse beträgt das Verhältnis 17βHydroxy-Steroid : 17α-Hydroxy-Steroid = 95 : 5.
Nach Aufarbeitung und Umkristallisation aus Methanol erhält man 14α,15α-Methylen-17β-estradiol-3-methylether: Fp.123 -127 °C.

### Beispiel 7

### 14α,15α-Methylen-17β-estradiol-3-methylether

Entsprechend Beispiel 6 wird 14α,15α-Methylen-estronmethylether mit Boran in Gegenwart des sich vom 1,2-Aminoalkohol (*R*)-2-(Diphenylhydroxymethyl)pyrrolidin ableitenden Katalysators reduziert, wobei 5 Mol% statt 10 Mol% 1,2-Aminoalkohol eingesetzt wurden.
Laut HPLC-Analyse beträgt das Verhältnis 17βHydroxy-Steroid : 17α-Hydroxy-Steroid = 93 : 7.
Nach Aufarbeitung und Umkristallisation aus Methanol erhält man 14α,15α-Methylen-17β-estradiol-3-methylether: Fp.124 - 127 °C.

### Beispiel 8

### 14α,15α-Methylen-17β-estradiol-3-methylether

Entsprechend Beispiel 7 wird 14α,15α-Methylen-estronmethylether mit Boran in Gegenwart des sich vom 1,2-Aminoalkohol (*R*)-2-(Diphenylhydroxymethyl)pyrrolidin ableitenden Katalysators reduziert, wobei 5 Mol% statt 10Mol % 1,2-Aminoalkohol eingesetzt wurden und die Reduktion statt bei 30 °C bei 20 °C durchgeführt wurde.
Laut HPLC-Analyse beträgt das Verhältnis 17β-Hydroxy-Steroid : 17α-Hydroxy-Steroid = 98 : 2.
Nach Aufarbeitung und Umkristallisation aus Methanol erhält man 14α,15α-Methylen-17β-estradiol-3-methylether: Fp.122 - 126 °C.

### Beispiel 9

### 14α,15α-Methylen-17β-estradiol-3-methylether

Entsprechend Beispiel 8 wird 14α, 15α-Methylen-estronmethylether mit Boran in Gegenwart des sich vom 1,2-Aminoalkohol (*R*)-2-(Diphenylhydroxymethyl)pyrrolidin ableitenden Katalysators reduziert, wobei 2.5 Mol% statt 10 Mol% 1,2-Aminoalkohol eingesetzt wurden und die Reduktion statt bei 30 °C bei 20 °C durchgeführt wurde.
Laut HPLC-Analyse beträgt das Verhältnis 17β-Hydroxy-Steroid : 17a-Hydroxy-Steroid = 96 : 4.
Nach Aufarbeitung und Umkristallisation aus Methanol erhält man 14α,15α-Methylen-17β-estradiol-3-methylether: Fp.120 -123 °C.

### Beispiel 10

### 14α,15α-Methylen-17β-estradiol-3-methylether

Entsprechend Beispiel 6 wird 14α, 15α-Methylen-estronmethylether mit Boran in Gegenwart des sich vom 1,2-Aminoalkohol (*R*)-2-(Diphenylhydroxymethyl)pyrrolidin ableitenden Katalysators reduziert, wobei die Reduktion statt bei 30 °C bei 40 °C durchgeführt wurde.
Laut HPLC-Analyse beträgt das Verhältnis 17β-Hydroxy-Steroid : 17α-Hydroxy-Steroid = 94 : 6.
Nach Aufarbeitung und Umkristallisation aus Methanol erhält man 14α,15α-Methylen-17β-estradiol-3-methylether: Fp.124 - 127 °C.

### Beispiel 11

### 14α,15α-Methylen-17β-estradiol-3-methylether

Entsprechend Beispiel 10 wird 14α,15α-Methylen-estronmethylether mit Boran in Gegenwart des sich vom 1,2-Aminoalkohol (*R*)-2-(Diphenylhydroxymethyl)pyrrolidin ableitenden Katalysators reduziert, wobei die Reduktion statt bei 30 °C bei 40 °C durchgeführt wurde und 5 Mol% statt 10 Mol %1,2-Aminoalkohol eingesetzt wurden.
Laut HPLC-Analyse beträgt das Verhältnis 17β-Hydroxy-Steroid : 17α-Hydroxy-Steroid = 94 : 6.
Nach Aufarbeitung und Umkristallisation aus Methanol erhält man 14α,15α-Methylen-17β-estradiol-3-methylether: Fp.125 - 127 °C.

### Beispiel 12

### 14α,15α-Methylen-17β-estradiol-3-methylether

Entsprechend Beispiel 1 wird 14α,15α-Methylen-estronmethylether mit Boran in Gegenwart des sich vom 1,2-Aminoalkohol (*R*)-Diphenyl-(3-thiazolin-4-yl)methanol ableitenden Katalysators reduziert, wobei die Reduktion statt bei 30 °C bei 65 °C durchgeführt wurde.
Laut HPLC-Analyse beträgt das Verhältnis 17β-Hydroxy-Steroid : 17α-Hydroxy-Steroid = 87 : 13.
Nach chromatographischer Reinigung und Umkristallisation aus Methanol erhält man 14α, 15α-Methylen-17β-estradiol-3-methylether: Fp.121 -123 °C.

### Beispiel 13

### 14α,15α-Methylen-17β-estradiol-3-methylether

Entsprechend Beispiel 1 wird 14α,15α-Methylen-estronmethylether mit Boran in Gegenwart des sich von 2-Aminoethanol ableitenden Katalysators reduziert.
Laut HPLC-Analyse beträgt das Verhältnis 17β-Hydroxy-Steroid : 17a-Hydroxy-Steroid = 77 : 23.
Nach chromatographischer Reinigung und Umkristallisation aus Methanol erhält man 14α,15α-Methylen-17β-estradiol-3-methylether: Fp.122,5 - 125,5 °C.

### Beispiel 14

### 14α,15α-Methylen-17β-estradiol-3-methylether

Entsprechend Beispiel 1 wird 14α,15α-Methylen-estronmethylether mit Boran in Gegenwart des sich vom 1,2-Aminoalkohol (*RS*)-2-Amino-1,1-diphenyl-3-(isopropylsulfanyl) propan-1-ol ableitenden Katalysators reduziert.
Laut HPLC-Analyse beträgt das Verhältnis 17β-Hydroxy-Steroid : 17α-Hydroxy-Steroid = 69 : 31.
Nach chromatographischer Reinigung und Umkristallisation aus Methanol erhält man 14α,15α-Methylen-17β-estradiol-3-methylether: Fp.124 - 127,5 °C.

### Beispiel 15

### 14α,15α-Methylen-17β-estradiol-3-methylether

Entsprechend Beispiel 8 wird wiederholt mit dem einzigen Unterschied, daß statt (*R*)-2-(Diphenylhydroxymethyl)pyrrolidin der 1,2-Aminoalkohol (*R*)-2-(Diphenylhydroxy-methyl)azetidin eingesetzt wurde.
Laut HPLC-Analyse beträgt das Verhältnis 17β-Hydroxy-Steroid : 17α-Hydroxy-Steroid = 99 : 1.
Nach Aufarbeitung und Umkristallisation aus Methanol erhält man 14α,15α-Methylen-17β-estradiol-3-methylether: Fp.123,5 - 127 °C.

### Beispiel 16

### 14α,15α-Methylen-17β-estradiol-3-methylether

Entsprechend Beispiel 1 wird 14α,15α-Methylen-estronmethylether mit Boran in Gegen-wart des sich vom 1,2-Aminoalkohol (*R*)-(1,2,3,4-Tetrahydroisochinolin-3-yl)methanol ableitenden Katalysators reduziert.
Laut HPLC-Analyse beträgt das Verhältnis 17β-Hydroxy-Steroid : 17α-Hydroxy-Steroid = 95 : 5.
Nach Aufarbeitung und Umkristallisation aus Methanol erhält man 14α,15α-Methylen-17β-estradiol-3-methylether: Fp.122 - 124,5 °C.

### Beispiel 17

### 14α,15α-Methylen-17β-estradiol-3-methylether

Entsprechend Beispiel 1 wird 14α,15α-Methylen-estronmethylether mit Boran in Gegenwart des sich vom 1,2-Aminoalkohol (*R,R*)-2,2'-Ethylendiiminobis(1-butanol) ableitenden Katalysators reduziert.
Laut HPLC-Analyse beträgt das Verhältnis 17β-Hydroxy-Steroid : 17α-Hydroxy-Steroid = 73 : 27.
Nach chromatographischer Reinigung und Umkristallisation aus Methanol erhält man 14α,15α-Methylen-17β-estradiol-3-methylether: Fp.123 - 126 °C.

### Beispiel 18

### 14α,15α-Methylen-17α-estradiol-3-methylether

Entsprechend Beispiel 1 wird 14α,15α-Methylen-estron-3-methylether mit Boran in Gegenwart des sich vom 1,2-Aminoalkohol (*S*)-2-(Diphenylhydroxymethyl)azetidin ableitenden Katalysators reduziert.
Laut HPLC-Analyse beträgt das Verhältnis 17β-Hydroxy-Steroid : 17α-Hydroxy-Steroid = 22 : 78.
Nach chromatographischer Reinigung und Umkristallisation aus Methanol erhält man 14α,15α-Methylen-17α-estradiol-3-methylether: Fp.146 - 147 °C.

### Beispiel 19

### 14α,15α-Methylen-17α-estradiol-3-methylether

Entsprechend Beispiel 1 wird 14α,15α-Methylen-estron-3-methylether mit Boran in Gegenwart des sich vom 1,2-Aminoalkohol (*S*)-2-(Diphenylhydroxymethyl)pyrrolidin ableitenden Katalysators reduziert.
Laut HPLC-Analyse beträgt das Verhältnis 17β-Hydroxy-Steroid : 17α-Hydroxy-Steroid = 14 : 86.
Nach chromatographischer Reinigung und Umkristallisation aus Methanol erhält man 14α,15α-Methylen-17α-estradiol-3-methylether: Fp.145 - 146,5 °C.

### Beispiel 20

### 14α,15α-Methylen-17α-estradiol-3-methylether

Entsprechend Beispiel 1 wird 14α,15α-Methylen-estron-3-methylether mit Boran in Gegenwart des sich vom 1,2-Aminoalkohol (*R*)-2-Amino-1,1-diphenyl-3-(isopropylsulfanyl)propan-1-ol ableitenden Katalysators reduziert.
Laut HPLC-Analyse beträgt das Verhältnis 17β-Hydroxy-Steroid: 17α-Hydroxy-Steroid = 29 : 80.
Nach chromatographischer Reinigung und Umkristallisation aus Methanol erhält man 14α,15α-Methylen-17α-estradiol-3-methylether: Fp.145 - 147 °C.

### Beispiel 21

### 14α,15α-Methylen-17α-estradiol-3-methylether

Entsprechend Beispiel 1 wird 14α,15α-Methylen-estron-3-methylether mit Boran in Gegenwart des sich vom 1,2-Aminoalkohol (*1'S*,*2'S*)-1-(1'-Amino-2'-methylbutyl)cyclopentanol ableitenden Katalysators reduziert.
Laut HPLC-Analyse beträgt das Verhältnis 17β-Hydroxy-Steroid : 17α-Hydroxy-Steroid = 22 : 78.
Nach chromatographischer Reinigung und Umkristallisation aus Methanol erhält man 14α,15α-Methylen-17α-estradiol-3-methylether: Fp.144 - 146 °C.

### Beispiel 22

### 14α,15α-Methylen-17α-estradiol-3-methylether

Entsprechend Beispiel 1 wird 14α,15α-Methylen-estron-3-methylether mit Boran in Gegenwart des sich vom 1,2-Aminoalkohol (*R*)-(1,1-Dioxo-[1,2]thiazetidin-3-yl)diphenyl-methanol ableitenden Katalysators reduziert.
Laut HPLC-Analyse beträgt das Verhältnis 17β-Hydroxy-Steroid : 17α-Hydroxy-Steroid = 33 : 67.
Nach chromatographischer Reinigung und Umkristallisation aus Methanol erhält man 14α,15α-Methylen-17α-estradiol-3-methylether: Fp.143 - 146 °C.

### Beispiel 23

### 3-Methoxy-14α,15α-methylen-estra-1,3,5(10),8-tetraen-17β-ol

Entsprechend Beispiel 1 wird 3-Methoxy-14α,15α-methylen-estra-1,3,5(10),8-tetraen-17-on mit Boran in Gegenwart des sich vom 1,2-Aminoalkohol (*R*)-1-(1'-Amino-1'-phenylmethyl)cyclopentanol ableitenden Katalysators reduziert.
Laut HPLC-Analyse beträgt das Verhältnis 17β-Hydroxy-Steroid : 17α-Hydroxy-Steroid = 98 : 2
Nach Aufarbeitung und Umkristallisation aus Methanol erhält man 3-Methoxy-14α,15α-methylen-1,3,5(10),8-tetraen-17β-ol: Fp. 135 - 137 °C.

### Beispiel 24

### 14-Dehydro-17α-estradiol-3-methylether

Entsprechend Beispiel 1 wird 14-Dehydro-estron-methylether mit Boran in Gegenwart des sich vom 1,2-Aminoalkohol (*S*)-2-(Diphenylhydroxymethyl)-azetidin ableitenden Katalysators reduziert.
Laut HPLC-Analyse beträgt das Verhältnis 17α-Hydroxy-Steroid : 17β-Hydroxy-Steroid = 92 : 8.
Nach Aufarbeitung und Umkristallisation aus Ether/Petrolether erhält man 14-Dehydro-17α-estradiol-3-methylether: Fp. 105 - 108 °C.

### Beispiel 25

### 14-Dehydro-17α-estradiol-3-methylether

Entsprechend Beispiel 1 wird 14-Dehydro-estron-methylether mit Boran in Gegenwart des sich vom 1,2-Aminoalkohol (*S*)-2-(Diphenyl-hydroxymethyl)pyrrolidin ableitenden Katalysators reduziert.
Laut HPLC-Analyse beträgt das Verhältnis 17α-Hydroxy-Steroid : 17β-Hydroxy-Steroid = 77 : 23.
Nach Aufarbeitung und Umkristallisation aus Ether/Petrolether erhält man 14-Dehydro-17α-estradiol-3-methylether: Fp. 106 - 108 °C.

### Beispiel 26

### 14-Dehydro-17α-estradiol-3-methylether

Unter Argonatmosphäre werden 0,15 mmol (10 Mol%) des sich vom 1,2-Aminoalkohol (*S*)-2-(Diphenylhydroxymethyl)-azetidin ableitenden 1,3,2-Oxazaborolidins in 4 ml Toluol gelöst und auf 30 °C erwärmt. 1,5 mmol 14-Dehydro-estron-3-methylether werden gelöst in 3 ml Toluol zugegeben. Zu dieser Lösung tropft man 0,36 g (3 mmol) Catecholboran in 3 ml Toluol über einen Zeitraum von 20 min zu.
Der Umsatz wird dünnschichtchromatographisch kontrolliert.
Nach vollständiger Reaktion gibt man zur Hydrolyse 15 ml Wasser zu. Die Toluolphase wird dreimal mit je 20 ml 2N NaOH ausgeschüttelt, um Catecholboran abzutrennen und dreimal mit jeweils 15 ml 2N HCI gewaschen, wobei der Katalysator als Hydrochlorid in die wäßrige Phase übergeht. Nach dem Ausschütteln mit ges. NaCl-Lösung wird die organische Phase mit Magnesiumsulfat getrocknet und im Rotationsverdampfer vollständig eingeengt.
Laut HPLC-Analyse beträgt das Diastereomerenverhältnis 17α-Hydroxy-Steroid : 17β-Hydroxy-Steroid = 76 : 24.
Nach Aufarbeitung und Umkristallisation aus Ether/Petrolether erhält man 14-Dehydro-17α-estradiol-3-methylether: Fp. 105 - 108 °C.

### Beispiel 27

### 14-Dehydro-17α-estradiol-3-methylether

Entsprechend Beispiel 27 wird 14-Dehydro-estron-3-methylether reduziert, wobei die Reduktion statt bei 30 °C bei -15 °C durchgeführt wurde.
Laut HPLC-Analyse beträgt das Verhältnis 17α-Hydroxy-Steroid : 17β-Hydroxy-Steroid = 75 : 25.
Nach Aufarbeitung und Umkristallisation aus Ether/Petrolether erhält man 14-Dehydro-17α-estradiol-3-methylether: Fp. 105 - 108 °C.

### Beispiel 28

### 14-Dehydro-17α-estradiol-3-methylether

Entsprechend Beispiel 27 wird 14-Dehydro-estron-3-methylether reduziert, wobei 20 Mol% statt 10 Mol% des sich vom 1,2-Aminoalkohol (S)-2-(Diphenylhydroxymethyl)-azetidin ableitenden 1,3,2,-Oxazaborolidin eingesetzt wurden.
Laut HPLC-Analyse beträgt das Verhältnis 17α-Hydroxy-Steroid : 17β-Hydroxy-Steroid = 72 : 28.
Nach Aufarbeitung und Umkristallisation aus Ether/Petrolether erhält man 14-Dehydro-17α-estradiol-3-methylether: Fp. 105 - 107 °C.

### Beispiel 29

### 14-Dehydro-17α-estradiol-3-methylether

Entsprechend Beispiel 27 wird 14-Dehydro-estron-3-methylether reduziert, wobei die Reduktion statt bei 30 °C bei 20 °C durchgeführt wurde. Laut HPLC-Analyse beträgt das Verhältnis 17α-Hydroxy-Steroid : 17β-Hydroxy-Steroid = 71 : 29.
Nach Aufarbeitung und Umkristallisation aus Ether/Petrolether erhält man 14-Dehydro-17α-estradiol-3-methylether: Fp. 106 - 108 °C.

### Beispiel 30

### 14-Dehydro-17α-estradiol-3-methylether

Entsprechend Beispiel 27 wird 14-Dehydro-estron-3-methylether reduziert, wobei die Reduktion statt bei 30 °C bei 0 °C durchgeführt wurde. Laut HPLC-Analyse beträgt das Verhältnis 17α-Hydroxy-Steroid : 17β-Hydroxy-Steroid = 80 : 20.
Nach Aufarbeitung und Umkristallisation aus Ether/Petrolether erhält man 14-Dehydro-17α-estradiol-3-methylether: Fp. 106 - 107 °C.

### Beispiel 31

### 14-Dehydro-17α-estradiol-3-methylether

Entsprechend Beispiel 27 wird 14-Dehydro-estron-3-methylether mit Catecholboran in Gegenwart des sich vom 1,2-Aminoalkohol (*S*)-1-(1 -Amino-1 -phenylmethyl)cyclopentanol ableitenden Katalysators reduziert, wobei die Reduktion statt bei 30 °C bei 20 °C durchgeführt wurde.
Laut HPLC-Analyse beträgt das Verhältnis 17α-Hydroxy-Steroid : 17β-Hydroxy-Steroid = 46 : 54.
Nach Aufarbeitung und Umkristallisation aus Ether/Petrolether erhält man 14-Dehydro-17α-estradiol-3-methylether: Fp. 105 - 107 °C.

### Beispiel 32

### 14-Dehydro-17α-estradiol-3-methylether

Entsprechend Beispiel 27 wird 14-Dehydro-estron-3-methylether mit Catecholboran in Gegenwart des sich vom 1,2-Aminoalkohol [(1*R*, 3*S*, 5*R*,1 *S*)-2-Azabicyclo[3.3.0]octan-3-yl]-(1 -p-tolyl)methanol ableitenden Katalysators reduziert, wobei die Reduktion statt bei 30 °C bei 20 °C durchgeführt wurde.
Laut HPLC-Analyse beträgt das Verhältnis 17α-Hydroxy-Steroid : 17β-Hydroxy-Steroid = 44 : 56.
Nach Aufarbeitung und Umkristallisation aus Ether/Petrolether erhält man 14-Dehydro-17α-estradiol-3-methylether: Fp. 105 - 106 °C.

## Patentansprüche

1. Verfahren zur stereoselektiven Reduktion von 17-Oxo-Steroiden der allgemeinen Formel I 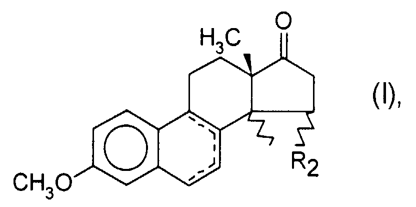 in der das Symbol 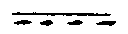 eine Einfach- oder eine Doppelbindung bedeutet und
R₂ entweder eine CH₂-Gruppe oder eine weitere Bindung zwischen den C-Atomen 14 und 15 darstellt, zu entsprechenden 17α- oder 17β-Alkoholen,
dadurch gekennzeichnet,
daß man die Reduktion mit Boran oder Catecholboran in Gegenwart von homochiralen 1,2-Aminoalkoholen-Katalysatoren durchführt.

2. Verfahren nach Anspruch 1,
dadurch gekennzeichnet, daß die Konzentration des Katalysators 0,1 bis 30 Mol%, bezogen auf das betreffende 17-Oxo-Steroid beträgt.

3. Verfahren nach Anspruch 1 und 2,
dadurch gekennzeichnet, daß man den Katalysator in einer Konzentration von 5 bis 10 Mol% einsetzt.

4. Verfahren nach Anspruch 1,
dadurch gekennzeichnet, daß man die Reduktion bei Temperaturen zwischen -30 und + 70 °C durchführt.

5. Verfahren nach Anspruch 1 und 4,
dadurch gekennzeichnet, daß man die Reduktion bei Temperaturen zwischen +10 und + 40 °C durchführt.

## Claims

1. Method for the stereoselective reduction of 17-oxo-steroids corresponding to the general formula I 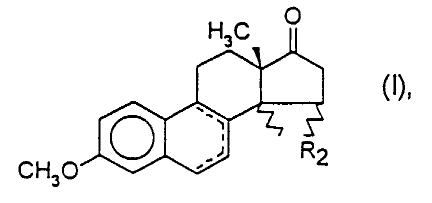 wherein the symbol 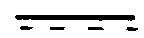 denotes a single bond or a double bond and
R₂ is either a CH₂- group or an additional bond between the C atoms 14 and 15,
to corresponding 17α- or 17β- alcohols,
characterised in that
the reduction is carried out using borane or catecholborane in the presence of homochiral 1,2-aminoalcohol catalysts.

2. Method according to claim 1,
characterised in that the concentration of the catalyst is 0.1 to 30 mol%, based on the relevant 17-oxosteroid.

3. Method according to claims 1 and 2,
characterised in that the catalyst is used in a concentration of from 5 to 10 mol%.

4. Method according to claim 1,
characterised in that the reduction is carried out at temperatures of between -30°C and +70°C.

5. Method according to claims 1 and 4,
characterised in that the reduction is carried out at temperatures of between +10°C and +40°C.

## Revendications

1. Procédé pour la réduction stéréosélective de 17-oxo-stéroïdes de la formule générale I 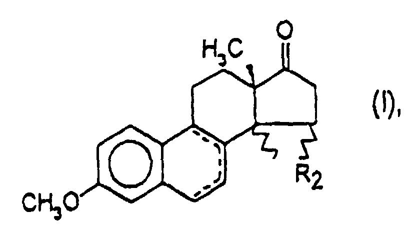 où le symbole 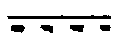 signifie une simple ou double liaison et
R₂ représente soit un groupe CH₂ ou une autre liaison entre les atomes C 14 et 15, en 17α ou 17β alcools correspondants,
caractérisé en ce que
on accomplit la réduction avec du borane ou du catécholborane en présence de catalyseurs de 1, 2-aminoalcools homochiraux.

2. Procédé selon la revendication 1,
caractérisé en ce que la concentration du catalyseur est de 0,1 à 30 % en moles en se rapportant au 17-oxo-stéroïde correspondant.

3. Procédé selon la revendication 1 et 2,
caractérisé en ce que l'on utilise le catalyseur à une concentration de 5 à 10 % en moles.

4. Procédé selon la revendication 1,
caractérisé en ce que l'on accomplit la réduction à des températures comprises entre - 30 et + 70°C.

5. Procédé selon la revendication 1 et 4,
caractérisé en ce que l'on accomplit la réduction à des températures comprises entre + 10 et + 40°C.
